# EUROPEAN PATENT SPECIFICATION

(11) **EP 2 327 353 B1**
(45) Date of publication and mention of the grant of the patent: **09.03.2016**
(21) Application number: 10014690.1
(22) Date of filing: 17.11.2010
(51) Int. Cl.: A61B 1/00

(54) **Endoscope cover fixing device and fixing system**
Endoskopabdeckungsbefestigungsvorrichtung und Befestigungssystem
Dispositif de fixation d'embout d'endoscope et système de fixation

(30) Priority: 30.11.2009 JP 2009271766
(43) Date of publication of application: 01.06.2011
(73) Proprietor: Covidien LP, Mansfield, MA 02048 (US)
(72) Inventor: Watanabe, Motonori, Fukuroi Shizuoka 437-0004 (JP); Iwamizu, Keita, Fukuroi Shizuoka 437-0004 (JP)
(74) Representative: Olsson, Carl H.S.

(56) References cited:
- EP-A2- 1 905 478
- WO-A2-2008/006114
- US-A- 4 369 794

## Description

### FIELD OF THE INVENTION

The present invention generally relates to an endoscope cover fixing device for, when a cover having a closed distal end is placed over an endoscope, fixing the position of the cover on the endoscope, and to a system employed for fixing the cover on the endoscope.

### BACKGROUND OF THE INVENTION

By necessity, medicine requires the diagnosis and treatment of interior body cavities that are not otherwise visible, and endoscopes are a well-known conventional medical instrument for implementing such diagnoses in a comparatively simple manner. Common endoscope structures comprise a lens portion provided in the distal end of a tube of a size that is insertable in an interior body cavity, and images captured by this lens portion are transmitted by way of optical fibers arranged within the tube.

Viscous body fluids and impurities affix to the outer circumferential surface of an endoscope during use. Accordingly, endoscopes must be thoroughly cleansed and disinfected after use to prevent the transfer of infectious diseases and the like. However, the complicated and time-consuming nature of the endoscope cleansing and disinfecting operations affects the operability thereof. A further problem pertains to the accelerated deterioration of the endoscope caused by the regular cleansing and disinfecting thereof.

With this in mind, the use of a sheath-like, contamination-preventing endoscope cover which has a closed distal end and which is placed over the endoscope to prevent contamination of the endoscope and is discarded after use has been recently considered.

However, the acquisition of satisfactory images with a cover placed over the endoscope requires that the cover be fitted closely to the lens portion in the distal end of the tube. However, with covers of this type, dimensional variations in the manufacturing process thereof are unavoidable. Such variations often render it difficult to fit the the cover closely to the lens portion of the tube.

With this in mind, structures for fixing a cover to an endoscope have been hitherto proposed (for example, see Japanese Patent No. 335932). In the cover-type endoscope as described in the '932 Japanese patent, an engagement portion is provided in the inner circumference of the proximal end side of the cover, and a slidable latch portion corresponding to the engagement portion is provided in the outer circumference of an operating portion of the endoscope. According to this configuration, friction between the latch portion and the engagement portion causes the latch portion to engage closely with the engagement portion which, in turn, facilitates fixing of the position of the cover on the endoscope.

However, the close engagement and fixing of a cover to an endoscope necessitates the employment of components that enhance the rigidity of the cover itself, and that increase the friction force in the conventional art as described above. The employment of these components increases the complexity of the machining and the assembly of the device, and also increases manufacturing and other costs.

Thereupon, the employment of a fixing device for positioning and fixing a cover to an endoscope configured separately to the endoscope and the cover such as a conventional medical clamp for example, was briefly considered. However, the employment of a conventional medical clamp comprises clamping the cover and the endoscope together with a pair of clamping portions and locking them in the clamped state. However, there is a significant pressing force or pressure exerted on the endoscope in the radial direction. This increases the likelihood that the image guide or light guide of the endoscope, usually constituted from optical fibers, will be damaged.

WO 2008/006114 A2 discloses an endoscope to which a sleeve may be attached using a sealing device comprising a split clamp or a split clip with the features of the pre-characterising portion of claim 1.

### SUMMARY OF THE INVENTION

With the foregoing conditions in mind, it is an object of the present invention to provide an endoscope cover fixing device and fixing system able to reliably fix the position of a cover onto an endoscope without associated endoscope damage.

The present invention provides an endoscope cover fixing device in accordance with claim 1 and an endoscope cover fixing system in accordance with claim 4.

In general, this invention is directed to an endoscope cover fixing device employed for, when a cover having a closed distal end is placed over an endoscope, fixing the position of the cover on the endoscope. The device comprises a fixing device main body that has a bent section deformable in the direction in which the bend amount thereof increases. The device further comprises an insert portion into which the endoscope is insertable. The device further comprises a pair of fixing protrusion portions that have a contact surface and that are provided to protrude from a concave surface side of the aforementioned fixing device main body. The pair of fixing protrusion portions are mutually oppositely disposed in two opposing regions on the two sides of the concave surface. The contact surfaces of the pair of fixing protrusion portions are disposed in a parallel positional relationship with a constant distance therebetween and in a parallel positional relationship to the longitudinal direction of the endoscope when the endoscope is inserted in the insert portion. The pair of protrusion portions move in parallel in opposing directions during the deformation of the fixing device main body to ensure that the contact surfaces, by way of the cover, contact opposing positions on the outer circumferential surface of the endoscope.

When deformation occurs in the direction in which the bend amount of the fixing device main body increases, the pair of (oppositely disposed) fixing protrusion portions move in parallel, opposing directions. At this time, the contact surfaces of the pair of protrusion portions, with the gap distance therebetween maintained constant, contact opposing positions on the outer circumferential surface of the endoscope by way of the cover. Accordingly, during the fixing operation, a constant pressing force is exerted in the radial direction of the endoscope, and the exertion of an excess pressing force thereon is avoided. As a result, the cover is able to be reliably fixed in position on the endoscope without associated endoscope damage.

According to an embodiment of the invention, the endoscope cover fixing device is characterized in that the region across which the contact surfaces contact the outer circumferential surface of the endoscope by way of the cover extends linearly along the longitudinal direction of the aforementioned endoscope. The length of these regions of contact are established to be larger than the pitch of a coil disposed in an over-tube of the endoscope. Because the contact region of the contact surfaces extends lengthwise along the longitudinal direction of the endoscope, the pressing force is dispersed rather than concentrated on a specific region. As a result, damage to the endoscope is able to be more reliably prevented.

According to another embodiment of the invention, the endoscope cover fixing device is characterized by the provision of two open ends in the aforementioned fixing device main body, the provision in the two open ends of latch portions which latch together to maintain a deformed state, and the provision of a contact surface distance-maintaining mechanism for preventing the distance between the contact surfaces from increasing as a result of the displacement of the pair of fixing protrusion portions in the deformed state. The deformed state is maintained by the latch portions, and an increase in the distance between the contact surfaces is prevented by the contact surface distance-maintaining mechanism. Accordingly, a constant pressing force is maintained during the positional fixing operation and, as a result, the cover is able to be even more reliably positionally fixed to the endoscope.

Aspects of an embodiment of the invention are further directed to an endoscope cover fixing system for, when a cover having a closed distal end is placed over an endoscope, employing a fixing device to fix the position of the cover on the endoscope. The endoscope cover fixing system comprises a pair of fixing protrusion portions that have contact surfaces and a parallel positional relationship. The contact surfaces contact opposing positions on the outer circumferential surface of the endoscope by way of the cover with a constant distance maintained between the contact surfaces. As a result, a constant pressing force is exerted during the fixing operation while the position of the cover is fixed. Because the contact surfaces contact opposing positions on the outer circumferential surface of the endoscope by way of the cover with a constant distance maintained between the contact surfaces, a constant pressing force is exerted in the radial direction of the endoscope during the fixing operation, and the exertion of an excessive pressing force thereon is avoided. Accordingly, the position of the cover is reliably fixed to the endoscope without associated endoscope damage.

Other objects and features of embodiments of the invention will be in part apparent and in part pointed out hereinafter.

### BRIEF DESCRIPTION OF THE DRAWINGS

FIG. 1 is a schematic diagram of an endoscope cover fixing device, an endoscope and a cover of a first embodiment of the present invention;
FIG. 2(a) is a front view of the endoscope cover fixing device of FIG. 1 in an open state;
FIG.2(b) is a cross-sectional view taken along the line A--A of FIG. 2(a);
FIG. 3(a) is a left-side view of the endoscope cover fixing device (open state) of FIG. 2(a);
FIG. 3(b) is a right-side view of the endoscope cover fixing device (open state) of FIG. 2(a);
FIG. 4 (a) is a front view of the endoscope cover fixing device of FIG. 1 in a closed state;
FIG. 4(b) is a cross-sectional view along the line B--B of FIG. 4(a);
FIG. 5(a) is an enlarged, fragmentary cross-sectional view of the endoscope cover fixing device (open state) of FIG. 2(a) with the position of the cover fixed on the endoscope;
FIG. 5(b) is an enlarged, fragmentary cross-sectional view of the endoscope cover fixing device (closed state) of FIG. 4(a);
FIG. 6(a) is a front view of the endoscope cover fixing device (open state) of another embodiment of the present invention;
FIG. 6(b) is a right-side view of the endoscope cover fixing device (open state) of FIG. 6(a); and
FIG. 7 is a cross-sectional view of an endoscope cover fixing device (open state) of yet another embodiment of the present invention.

### REFERENCE CHARACTERS

11 Endoscope Set
21 Cover
22 Distal end of cover-tube
23 Proximal end of cover-tube
31 Endoscope
32 Cover-tube
33 Coil
41,81,91 Endoscope cover fixing device
42 Fixing device main body
43 Curved portion constituting bent section
44 First arm portion
45 Second arm portion
46 (First) open end
47 (Second) open end
51 Curved portion
52 Tubular portion
53 Latch release portion
54,57 Insert hole constituting insert portion
55 Concave surface
61,62 Fixing protrusion portion
61a,62a Contact surfaces
65a (First) latch portion
65b (Second) latch portion
82 Groove from which contact surface distance-maintaining mechanism is constituted
83 Protrusion from which contact surface distance-maintaining mechanism is constituted
92,93 Protruding piece from which contact surface distance-maintaining mechanism is constituted
C1 Longitudinal direction of endoscope
L1 Distance
P1 Pitch
R1 (Contact) region

Corresponding reference characters indicate corresponding parts throughout the drawings.

### DESCRIPTION OF THE PREFERRED EMBODIMENTS

An endoscope cover fixing device able to reliably fix the position of a cover on an endoscope without associated endoscope damage is provided.

### [First Embodiment]

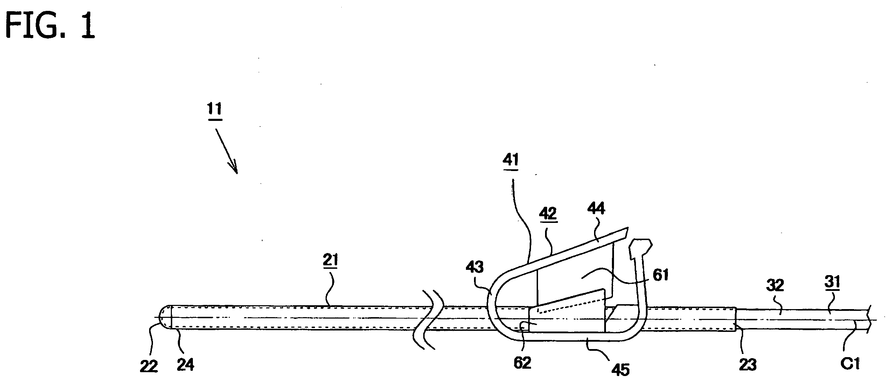

An endoscope cover fixing device of a first embodiment of the present invention will be hereinafter described with reference to FIGS. 1 to 5.

FIG. 1 schematically shows an endoscope set 11 comprising an endoscope cover fixing device 41, an endoscope 31 and an endoscope cover 21 of the first embodiment. The endoscope 31 comprises a lens portion (not shown) provided at a distal end 22 of a flexible cover tube 32. Tube 32 has a diameter suitable for insertion into a body cavity interior. In a non-limiting embodiment, the endoscope 31 has a maximum diameter of 0.5 mm, and is generally on the order of 0.4 mm. The endoscope cover 21 constitutes a sheath-like member formed from a flexible and light-permeable material such as a synthetic resin, and has a slightly larger internal diameter than the diameter of the endoscope 31. The distal end 22 of the endoscope cover 21 is rounded and closed in the illustrated embodiment. The cover 21 is placed over the endoscope 31 and, in this state, the inner surface of the distal end 22 of the cover 21 is fitted closely to the lens portion.

The endoscope cover fixing device 41, which constitutes a member for fixing the position of the cover 21 on the endoscope 31, is mounted in proximity of a proximal end 23 of the cover 21.

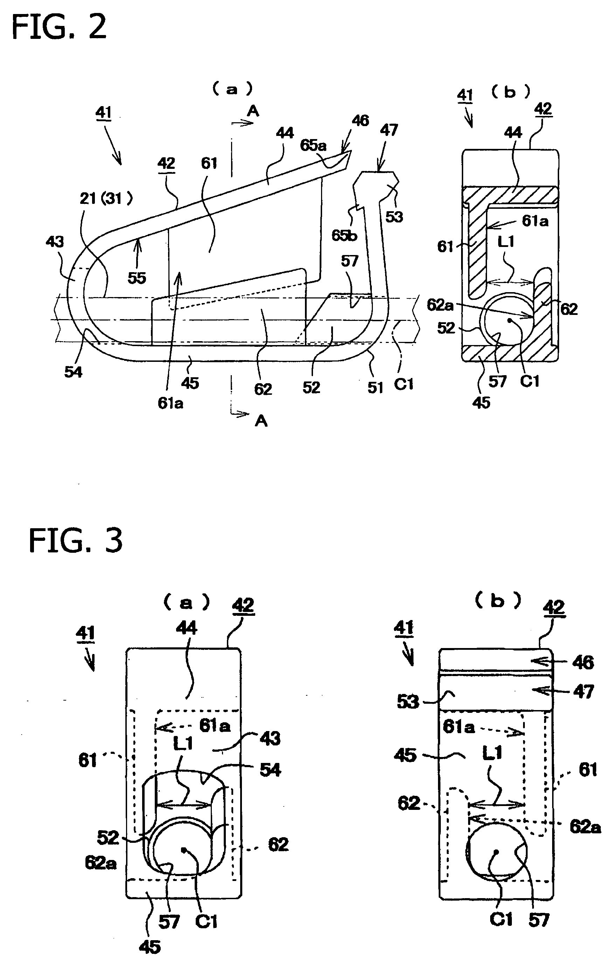

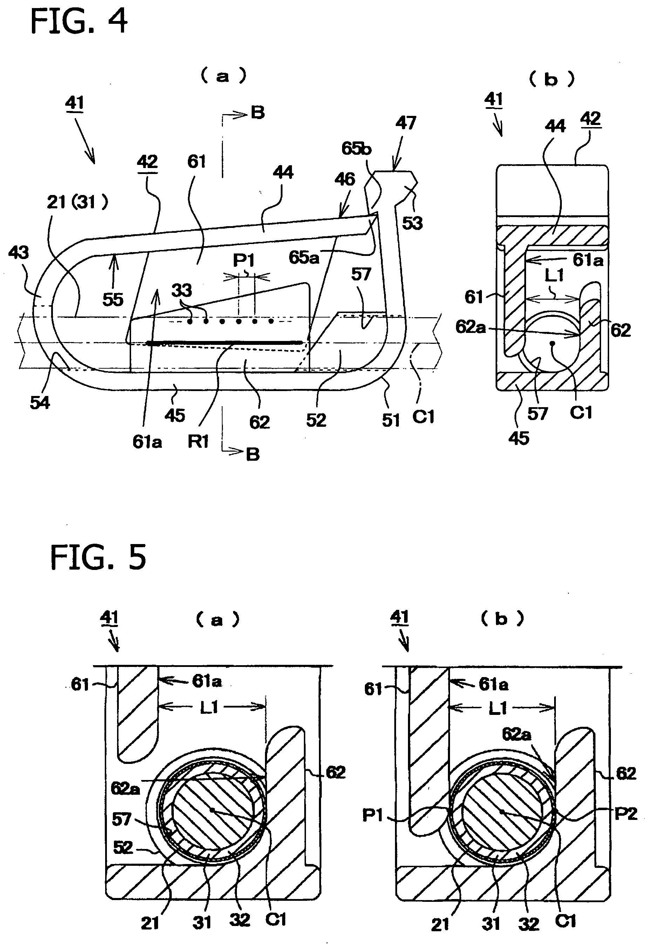

As illustrated in FIG. 1 and FIG. 2, the endoscope cover fixing device 41 includes a fixing device main body 42. The fixing device main body 42 is fabricated as a plate-like member of uniform width curved in an imperfect ring shape. The fixing device main body 42 includes a curved portion (bent section) 43 that bends at an angle of the order of 170° with respect to the side of the cover 21 comprising the distal end 22 as best illustrated in FIG. 1. One side of this curved portion 43 forms a first arm portion 44 while the other side forms a second arm portion 45. An additional curved portion 51 is provided in the second arm portion 45 that bends at an angle (approximately 90°, for example) with respect to the second arm portion. As illustrated in FIG. 4 and so on, the fixing device main body 42 is deformable in the direction in which the bend angle of the curved portion 43 increases, or in other words, in a direction in which the first arm portion 44 approaches the second arm portion 45.

In the open state shown in FIG. 2, a clearance exists between a first open end 46 of the distal end of the first arm portion 44 and a second open end 47 of the distal end of the second arm portion 45. The end portion of the first open end 46 is cut diagonally to form a first latch portion 65a. On the other hand, a latch release portion 53, preferably of greater thickness than other sections of the fixing device main body 42, is provided in the second open end 47. The region of the latch release portion 53 facing the concave surface 55 side of the fixing device main body 42 forms a second latch portion 65b able to lock with the first latch portion 65a. The role of the first latch portion 65a and the second latch portion 65b is to latch together to maintain the deformed state of the fixing device main body 42.

As shown in FIG. 2 and in FIG. 3, insert holes 54, 57 (insert portions) through which the endoscope 31 with the cover 21 placed thereover is insertable are formed in two parts of the fixing device main body 42. The proximal end side insert hole 57 is formed to penetrate the curved portion 51. A tubular portion 52 that protrudes to the concave surface 55 side is provided in the fixing device main body 42 in a position corresponding to the insert hole 57. The inner diameter of the insert hole 57 is slightly larger than the outer diameter dimension of the cover 21. The distal end side insert hole 54 is formed in such a way as to penetrate a curved portion 43. The aperture of the insert hole 54 is slightly bigger than the aperture of the insert hole 57 so that the endoscope 31, in a state in which it is covered by the cover 21, is easily inserted. The area of the aperture of the insert hole 54 may be even greater than illustrated.

As shown in FIGS. 2-5, the endoscope cover fixing device 41 comprises a pair of fixing protrusion portions 61, 62 that protrude from the concave surface 55 side of the fixing device main body 42. One fixing protrusion portion 61 is provided on the first arm portion 44, while the other fixing protrusion portion 62 is provided on the second arm portion 45. Notably, as best shown in FIG. 3, the fixing protrusion portions 61, 62 are offset in opposing directions along the width of the fixing device main body 42. In other words, the pair of fixing protrusion portions 61, 62 are mutually oppositely disposed in two regions on opposing sides of the concave surface 55. The expression "mutually oppositely disposed" used here refers to the arrangement of the protrusion portions 61, 62 to extend in opposing directions, and to their arrangement in positions that do not lie on the same plane.

Preferably, and as shown in FIGS. 2-5, the pair of fixing protrusion portions 61, 62 are planar members of equal thickness. The shape of the fixing protrusion portion 61 of the first arm portion 44 when seen from the thickness direction approximates a parallelogram. The fixing protrusion portion 62 of the second arm portion 45 is slightly smaller than the fixing protrusion portion 61 of the first arm portion 44 and describes a trapezoidal shape when viewed in the thickness direction. Notably, the interior of the distal end portion as defined by the pair of fixing protrusion portions 61, 62 is of a rounded shape designed to minimize damage to the cover 21 and the endoscope 31.

Flat contact surfaces 61a, 62a are formed in the interior of the pair of fixing protrusion portions 61, 62, and are disposed to describe a parallel positional relationship with a constant distance L1 therebetween. Similarly, when the endoscope 31 is inserted in the insert holes 54, 57, the flat contact surfaces 61a, 62a describe a parallel positional relationship to a longitudinal direction C1 of the endoscope 31. Notably, the aforementioned distance L1 is established in such a way as to be no less than the outer diameter dimension of the endoscope 31, and no more than the outer diameter dimension of the cover 21. In addition, the distance L1 is kept constant and does not change between the open state as shown in FIGS 2 and 3 and the closed state as shown in FIG. 4.

The cover tube 32 of the endoscope 31 comprises a coil 33. The pitch P1 of the coil 33 in this embodiment is approximately 0.6 mm (see FIG. 4(a)). With these conditions in mind, the length of the pair of fixing protrusion portions 61, 62 along the longitudinal direction C1 of the endoscope 31 is formed in such a way as to be longer than the pitch P1 of the coil 33. In a preferred embodiment, the length of the pair of fixing protrusion portions 61, 62 along the longitudinal direction C1 of the endoscope 31 is established to be no less than ¼ of the total length of the endoscope cover fixing device 41, and at least several times that of the pitch P1. For this reason, a region R1 where the contact surfaces 61a, 62a contact the outer circumferential surface of the endoscope 31 by way of the cover 21 extends linearly along the longitudinal direction C1. The length of the contact region R1 is desirably several times greater than the pitch P1.

The fixing device main body 42 of the endoscope cover fixing device 41 of this embodiment hence does not comprise a pair of clamping portions as seen in conventional medical clamps. In other words, this embodiment disposes of structures that flatten and deform the endoscope 31.

The endoscope cover fixing device 41 may be manufactured by injection molding or the like of, for example, a synthetic resin material. In an embodiment, the fixing device main body 42, the tubular portion 52 and the pair of fixing protrusion portions 61, 62 from which the endoscope cover fixing device 41 is constituted are formed as one piece of a synthetic resin material. Provided the material is flexible, there are no particular restrictions to the synthetic resin material which can be used for the endoscope cover fixing device 41, and various materials, most notably including polypropylene, polycarbonate and nylon, may beemployed.

A method for using the endoscope cover fixing device 41 of this embodiment is described. Prior to the endoscope 31 being used, the cover 21 is placed over the endoscope 31. The inner surface of the distal end 22 of the cover 21 is disposed to fit closely against the lens portion. The fixing device 41 is prepared and set to the open state. The endoscope 31, in a state in which it is covered by the cover 21, is inserted into the insert holes 54, 57 through the distal sides thereof and the fixing device 41 is moved to a position in close proximity of the proximal end 23 of the cover 21 (see FIG. 1). Insertion of the endoscope 31 into the fixing device 41 may be performed prior to placement of the cover 21 over the endoscope 31. At this point, the endoscope 31 is disposed with respect to the fixing device 41 in a state in which it is covered by the cover 21 (i.e., it is disposed along the second arm portion 45).

With the fixing device 41 held in the fingers, pressure is applied thereto in such a way that the first arm portion 44 approaches the second arm portion 45. The fixing device main body 42 is caused to deform in a direction in which the bend amount of the curved portion 43 increases. Thereupon, the endoscope cover fixing device 41 of the open state as shown in FIGS. 2 and 3 is established in the closed state as shown in FIG. 4, and the first latch portion 46 and second latch portion 47 latch together to maintain the closed state. At this time, even though the pair of fixing protrusion portions 61, 62 move in parallel in mutually opposing directions, no marked change in the distance L1 between the contact surfaces 61a, 62a occurs (see FIGS. 5(a),(b)). With the distance L1 maintained to be constant in this way, the contact surfaces 61a, 62a contact opposing positions P1, P2 on the outer circumferential surface of the endoscope 31 by way of the cover 21, and this action completes the positional fixing operation.

Following the use of the endoscope 31, the latch release portion 53 of the endoscope cover fixing device 41 is operated to release the lock between the first latch portion 65a and the second latch portion 65b which, in turn, results in restoration to the initial unlocked state, or the open state. The fixing device main body 42 is elastically restored to its original shape, and the open state of the fixing device 41 as shown in FIGS 2 and 3 is established. As a result, the fixing device 41 is then removable. While the used fixing device 41 may be discarded together with the cover 21, it may be cleansed and disinfected, and used again in accordance with need.

The following benefits, among others, are achieved by the embodiment described above. In the fixing device 41 of this embodiment in the configuration described above, even when the bend amount during the positional fixing operation increases, a constant pressure force is exerted on the endoscope 31 in the radial direction. In contrast, when a conventional medical clamp is employed and a provisional positional fixing is established, the cover and the endoscope are fastenably clamped by a pair of clamping portions thereof and then locked in the clamped state so established. Accordingly, a large pressure force is exerted in the radial direction on the endoscope. Using the fixing device 41 of this embodiment in the current invention, the exertion of excessive pressure force to the endoscope 31 in a state in which it is covered by the cover 21 is avoided. As a result, the cover 21 is able to be reliably fixed in position on the endoscope 31 without associated damage.

Additionally, the contact region R1 afforded by the contact surfaces 61a, 62a of the fixing device 41 extends linearly along the longitudinal direction C1 of the endoscope 31. The length of this contact region R1 is established in such a way as to be larger than the pitch P1 of the coil 33 of the over-tube 32 of the endoscope 31. Accordingly, the pressing force exerted by the contact surfaces 61a, 62a is reliably dispersed rather than concentrated on a specific region of the endoscope 31. Accordingly, the damage to the endoscope 31 is able to be more reliably prevented.

### [Second Embodiment]

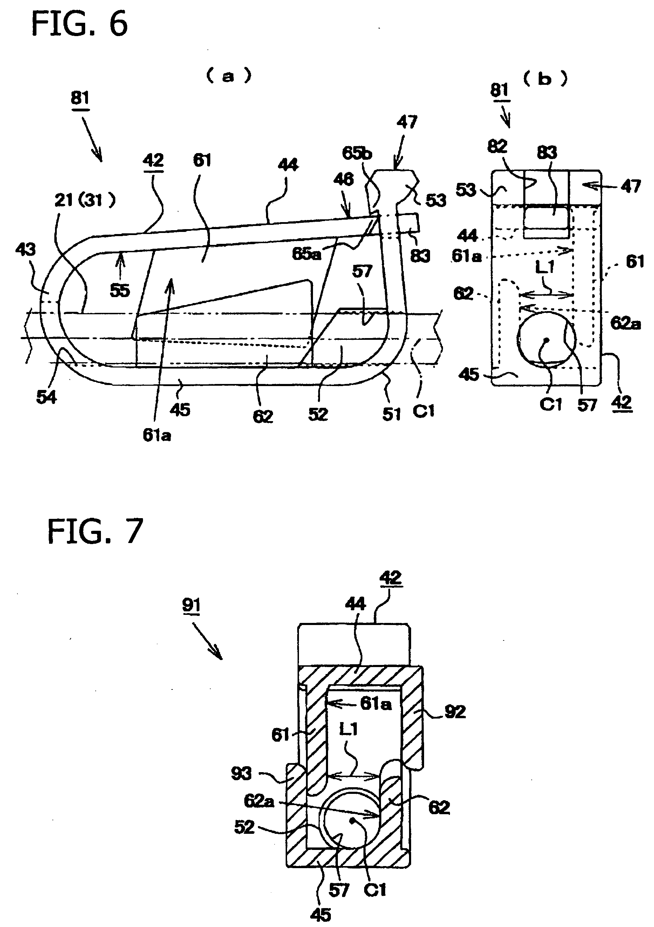

A fixing device 81 of a second embodiment of the present invention will be hereinafter described with reference to FIG. 6.

The fixing device 81 of this second embodiment differs from the first embodiment in that it additionally comprises a contact surface distance-maintaining mechanism. Specifically, a groove 82 from which the contact surface distance-maintaining mechanism is constituted is formed as a notch in the second open end 47 of the second arm portion 45 of the fixing device main body 42. A protrusion 83 from which the contact surface distance-maintaining mechanism is constituted is formed in the first open end 46 of the first arm portion 44 of the fixing device main body 42. The width of the protrusion 83 is slightly less than the width of the groove 82 and, as a result, the protrusion 83 can be inserted in the groove 82 during the deformation of the fixing device main body 42. As a result of the inability of the protrusion 83 to move in the width direction of the groove 82 at this time, displacement of the pair of fixing protrusion portions 61, 62 in the width direction of the fixing device main body 42 is prevented, and the distance L1 between the contact surfaces 61a, 62a is reliably maintained.

Accordingly, in this embodiment, a deformed state is maintained by the first latch portion 65a and second latch portion 65b, and an increase in the distance L1 between the contact surfaces 61a, 62a is prevented by the contact surface distance-maintaining mechanism. Because a constant pressing force is maintained during the positional fixing operation, the cover 21 is even more reliably fixed on the endoscope 31.

### [Third Embodiment]

A fixing device 91 of a third embodiment of the present invention will be hereinafter described with reference to FIG. 7.

The fixing device 91 of this third embodiment comprises a different type of contact surface distance-maintaining mechanism to the contact surface distance-maintaining mechanism of the second embodiment described above. A protrusion piece 92 from which the contact surface distance-maintaining mechanism is constituted is provided in a parallel, positional relationship to the fixed protrusion portion 61 in a position separate from the fixed protrusion portion 61 in the concave surface 55 side of the first arm portion 44. The inner surface side of this protrusion piece 92 is in slidable contact with the outer surface side of the other fixed protrusion portion 62. As a result, misalignment of the fixed protrusion portion 62 in the width direction is controlled. In addition, a protrusion piece 93 from which the contact surface distance-maintaining mechanism is constituted is provided in a parallel positional relationship to the fixed protrusion portion 62 in a position separate from the fixed protrusion portion 62 in the concave surface 55 side of the second arm portion 45 of the fixing device main body 42. The inner surface side of this protrusion piece 93 is in slidable contact with the outer surface side of the other fixed protrusion portion 61. As a result, misalignment of the fixed protrusion portion 61 in the width direction is controlled.

Accordingly, in this embodiment, the deformed shape is maintained by the first latch portion 65a and second latch portion 65b, and increase in the distance L1 between the contact surfaces 61a, 62a is prevented by the contact surface distance-maintaining mechanism. The maintenance of a constant pressing force during the positional fixing operation affords an even more reliable positional fixing of the cover 21 to the endoscope 31.

### [Other Embodiments]

Having described embodiments of the invention in detail, it will be apparent that modifications and variations are possible.

For example, while the bent section of the all embodiments is constituted from the curved portion 43, it may also be constituted from an elbow-shaped portion, or from other shapes.

The insert portion into which the endoscope 31 can be inserted in a state in which it is covered by a cover 21 is constituted by a hole that penetrates the fixing device main body 42. However, the configuration thereof is not limited in this manner and, for example, a notched portion or similar structure may also be adopted. The tubular portion 52 provided correspondence to the proximal end side insert hole 57, and may also or alternatively be omitted. In addition or in alternative, this tubular portion 52 may be provided corresponding to the distal end side insert hole 54.

While the aforementioned embodiments are produced as one piece of material by molding of the fixing device main body 42 and the pair of fixing protrusion portions 61, 62 from the same synthetic resin material, the pair of fixing protrusion portions 61, 62 may instead be molded separately from the fixing device main body 42 and joined by any suitable means (for example, adhesive-based adhesion or thermal welding). Although a synthetic resin material is selected as the material for moulding the fixing devices 41, 81, 91 of the aforementioned embodiments, a metal material such as stainless steel or aluminium may also be selected.

In other variations, a configuration in which the pair of protrusion portions 61, 62 of the fixing device are disposed in one of the two opposing regions of the concave surface 55 (i.e., either in the concave surface 55 side of the first arm portion 44 or the concave surface 55 side of the second arm portion 45) may be adopted. The parallel movement of the pair of fixing protrusion portions 61, 62 in the same direction as the direction in which the fixing device main body 42 is deformed allows the contact surfaces 61a, 62a to contact the aforementioned positions P1, P2 by way of the cover 21.

While the embodiments described above describe the employment of fixing devices 41, 81, 91 for fixing the position of the cover 21 on the medical endoscope 31, the application thereof in other medical instruments (including industrial endoscopes) is also possible.

## Claims

1. An endoscope cover fixing device (41), having an open state and a closed state, and comprising:
- a fixing device main body (42), having a concave surface (55) with a first region and with a second region opposing the first region, and comprising:
- a bent section (43) that is deformable in a direction in which a bend amount thereof increases, whereupon the endoscope cover fixing device (41) moves from its open state towards its closed state; and
- an insert portion (54, 57):
- into which an endoscope (31), having a longitudinal direction (C1) and covered by a sheath-like cover (21), is insertable;
**characterised in that**:
- the fixing device main body (42) further comprises:
- a first fixing protrusion portion (61) protruding from the first region and comprising a first contact surface (61a); and
- a second fixing protrusion portion (62) protruding from the second region and comprising a second contact surface (62a);
- the first fixing protrusion portion (61):
- extends in a direction opposing a direction of protrusion of the second fixing protrusion portion (62); and
- is arranged in a position that does not lie on a same plane as the second fixing protrusion portion (62);
- the first contact surface (61a) and the second contact surface (62a) are disposed:
- in a parallel positional relationship with a distance (L1) therebetween, which distance (L1) is the same in both the open state and the closed state; and
- in a parallel positional relationship to the longitudinal direction (C1) of the covered, insertable endoscope (31, 22), were such a covered, insertable endoscope (31, 22) to be inserted into said insert portion (54, 57);
whereby, when the endoscope cover fixing device (41) moves from its open state towards its closed state first and second contact surfaces (61a, 62a) move in parallel in opposing directions such that in the closed state, a contact region (R1) of the first contact surface (61a) contacts with a position on an outer circumferential surface of the inserted, covered endoscope (31, 22) opposing a position on the outer circumferential surface that is contacted by a contact region (R1) of the second contact surface (62a).

2. The endoscope cover fixing device (41) according to claim 1, configured for use with an insertable endoscope (31) comprising a cover tube (32), which comprises a coil (33) having a pitch (P1), wherein the contact regions (R1) extend linearly and are longer than the pitch (PI).

3. The endoscope cover fixing device (41) according to claim 1 or claim 2, wherein the fixing device main body (42) further comprises:
- two open ends (46, 47) comprising latch portions (65a, 65b) which lock together to maintain the closed state; and
- a contact surface distance-maintaining mechanism (82/83, 92/93) for preventing an increase in the distance (L1) between the contact surfaces (61a, 62a) .

4. An endoscope cover fixing system comprising:
- the endoscope cover fixing device (41) according to any of the preceding claims;
- the insertable endoscope (31); and
- the sheath-like cover (21);
wherein the distance (L1) is no less than an outer dimension of the insertable endoscope (31) and no more than an outer dimension of the sheath-like cover (21), whereby the sheath-like cover (21) may be fixed on the insertable endoscope (31) through exertion of a constant pressing force.

5. An endoscope cover fixing system according to claim 4, wherein the insertable endoscope (31) comprises a cover tube (32) comprising a coil (33).

## Patentansprüche

1. Endoskopabdeckungsbefestigungsvorrichtung (41) mit einem offenen Zustand und einem geschlossenen Zustand und umfassend:
- einen Hauptteil der Befestigungsvorrichtung (42), der eine konkave Oberfläche (55) mit einem ersten Bereich und mit einem zweiten Bereich aufweist, der dem ersten Bereich gegenüberliegt, und umfassend:
- einen gebogenen Abschnitt (43), der in einer Richtung verformbar ist, wobei sich sein Biegeanteil erhöht, woraufhin die Endoskopabdeckungsbefestigungsvorrichtung (41) von ihrem offenen Zustand in ihren geschlossenen Zustand wechselt; und
- einen Einführabschnitt (54, 57):
- in das ein Endoskop (31) mit einer Längsrichtung (C1) und mit einer mantelartigen Abdeckung (21) eingeführt werden kann;
**dadurch gekennzeichnet, dass**:
- der Hauptteil der Befestigungsvorrichtung (42) weiter umfasst:
- einen ersten vorstehenden Befestigungsabschnitt (61), der aus dem ersten Bereich herausragt und eine erste Kontaktfläche (61 a) umfasst; und
- einen zweiten vorstehenden Befestigungsabschnitt (62), der aus dem zweiten Bereich herausragt und eine zweite Kontaktfläche (62a) umfasst;
- der erste vorstehende Befestigungsabschnitt (61):
- in eine Richtung verläuft, die der Herausragerichtung des zweiten vorstehenden Befestigungsabschnitts (62) entgegengesetzt ist; und
- in einer Position angeordnet ist, die nicht in derselben Ebene wie der zweite vorstehende Befestigungsabschnitt (62) liegt;
- die erste Kontaktfläche (61 a) und die zweite Kontaktfläche (62a) angeordnet sind:
- in einer parallelen Lagebeziehung mit einem Abstand (L1) zwischen ihnen, wobei der Abstand (L1) sowohl im offenen Zustand als auch im geschlossenen Zustand gleich ist; und
- in einer parallelen Lagebeziehung zur Längsrichtung (C1) des abgedeckten, einführbaren Endoskops (31, 22), in der ein solches abgedecktes, einführbares Endoskop (31, 22) in den Einführabschnitt (54, 57) einzuführen ist;
wobei, wenn die Endoskopabdeckungsbefestigungsvorrichtung (41) von ihrem geöffneten Zustand in ihren geschlossenen Zustand wechselt, die erste und die zweite Kontaktfläche (61 a, 62a) sich parallel in entgegengesetzte Richtungen bewegen, so dass, im geschlossenen Zustand, ein Kontaktbereich (R1) der ersten Kontaktfläche (61 a) eine Stelle auf einer äußeren Umfangsfläche des eingeführten, abgedeckten Endoskops (31, 22) berührt, die gegenüber einer Stelle auf der äußeren Umfangsfläche liegt, die von einem Kontaktbereich (R1) der zweiten Kontaktfläche (62a) berührt wird.

2. Endoskopabdeckungsbefestigungsvorrichtung (41) nach Anspruch 1, ausgelegt für die Verwendung mit einem einführbaren Endoskop (31), umfassend ein Schutzrohr (32), das eine Spule (33) mit einem Wicklungsschritt (P1) umfasst, wobei die Kontaktbereiche (R1) linear verlaufen und länger als der Wicklungsschritt (P1) sind.

3. Endoskopabdeckungsbefestigungsvorrichtung (41) nach Anspruch 1 oder Anspruch 2, wobei der Hauptteil der Befestigungsvorrichtung (42) weiter umfasst:
- zwei offene Enden (46, 47), die Verriegelungsteile (65a, 65b) umfassen, die ineinandergreifen, um den geschlossenen Zustand aufrechtzuerhalten; und
- einen Mechanismus zur Aufrechterhaltung des Abstandes zwischen den Kontaktflächen (82/83, 92/93), um eine Vergrößerung des Abstandes (L1) zwischen den Kontaktflächen (61 a, 62a) zu verhindern.

4. Endoskopabdeckungsbefestigungssystem umfassend:
- die Endoskopabdeckungsbefestigungsvorrichtung (41) nach einem der vorstehenden Ansprüche;
- das einführbare Endoskop (31); und
- die mantelartige Abdeckung (21);
wobei der Abstand (L1) nicht kleiner als ein Außenmaß des einführbaren Endoskops (31) ist und nicht größer als ein Außenmaß der mantelartigen Abdeckung (21) ist, wobei die mantelartige Abdeckung (21) auf dem einführbaren Endoskop (31) durch Ausübung einer konstanten Anpresskraft befestigt werden kann.

5. Endoskopabdeckungsbefestigungssystem nach Anspruch 4, wobei das einführbare Endoskop (31) ein Schutzrohr (32) umfasst, das eine Spule (33) umfasst.

## Revendications

1. Dispositif de fixation de recouvrement d'endoscope (41) ayant un état ouvert et un état fermé, et comprenant :
- un corps principal de dispositif de fixation (42) ayant une surface concave (55) avec une première zone et avec une deuxième zone opposée à la première zone, et comprenant :
- un tronçon courbe (43) déformable dans une direction dans laquelle une quantité de courbure de celui-ci augmente, sur quoi le dispositif de fixation de recouvrement d'endoscope (41) se déplace de son état ouvert vers son état fermé ; et
- une partie d'insertion (54, 57) :
- dans laquelle il est possible d'insérer un endoscope (31) ayant une direction longitudinale (C1) et recouvert par un recouvrement semblable à une gaine (21), **caractérisé en ce que** :
- le corps principal du dispositif de fixation (42) comprend en outre :
- une première partie de fixation saillante (61) en saillie par rapport à la première zone et comprenant une première surface de contact (61a) ; et
- une deuxième partie de fixation saillante (62) en saillie par rapport à la deuxième zone et comprenant une deuxième surface de contact (62a) ;
- la première partie de fixation saillante (61) :
- s'étendant dans une direction opposée à la direction en saillie de la deuxième partie de fixation saillante (62) ; et
- étant disposée dans une position qui ne repose pas dans un même plan que la deuxième partie de fixation saillante (62) ;
- la première surface de contact (61a) et la deuxième surface de contact (62a) étant disposées :
- selon un rapport de positionnement parallèle avec une distance (L1) entre elles, laquelle distance (L1) est la même dans l'état ouvert tout comme dans l'état fermé ; et
- selon un rapport de positionnement parallèle à la direction longitudinale (C1) de l'endoscope (31, 22) insérable recouvert, où un tel endoscope (31, 22) insérable recouvert doit être inséré à l'intérieur de ladite partie d'insertion (54, 57) ;
moyennant quoi, lorsque le dispositif de fixation de recouvrement d'endoscope (41) se déplace de son état ouvert vers son état fermé, des première et deuxième surfaces de contact (61a, 62a) se déplacent en parallèle dans des directions opposées de telle sorte qu'à l'état fermé, une zone de contact (R1) de la première surface de contact (61a) entre en contact avec une position sur une surface circonférentielle extérieure de l'endoscope (31, 22) inséré recouvert en opposition avec une position sur la surface circonférentielle extérieure qui est contactée par une zone de contact (R1) de la deuxième surface de contact (62a).

2. Dispositif de fixation de recouvrement d'endoscope (41) selon la revendication 1, configuré pour l'utilisation avec un endoscope (31) insérable comprenant un tube de recouvrement (32), lequel comprend une bobine (33) ayant un pas (P1), dans lequel les zones de contact (R1) s'étendent linéairement et sont plus longues que le pas (P1).

3. Dispositif de fixation de recouvrement d'endoscope (41) selon la revendication 1 ou la revendication 2, dans lequel le corps principal du dispositif de fixation (42) comprend en outre :
- deux extrémités ouvertes (46, 47) comprenant des portions de verrouillage (65a, 65b) qui se verrouillent ensemble pour maintenir l'état fermé ; et
- un mécanisme de maintien de distance des surfaces de contact (82/83, 92/93) pour empêcher une augmentation de la distance (L1) entre les surfaces de contact (61a, 62a).

4. Système de fixation de recouvrement d'endoscope comprenant :
- le dispositif de fixation de recouvrement d'endoscope (41) selon l'une quelconque des revendications précédentes ;
- l'endoscope (31) insérable ; et
- le recouvrement (21) semblable à une gaine ;
dans lequel la distance (L1) n'est pas inférieure à une dimension extérieure de l'endoscope insérable (31) et n'est pas supérieure à une dimension extérieure du recouvrement (21) semblable à une gaine, moyennant quoi le recouvrement (21) semblable à une gaine peut être fixé sur l'endoscope insérable (31) grâce à l'exercice d'une force de pression constante.

5. Système de fixation de recouvrement d'endoscope selon la revendication 4, dans lequel l'endoscope insérable (31) comprend un tube de recouvrement (32) comprenant une bobine (33).
